# EUROPÄISCHE PATENTANMELDUNG

(11) **EP 1 878 434 A1**
(43) Veröffentlichungstag der Anmeldung: **16.01.2008**
(21) Anmeldenummer: 07012930.9
(22) Anmeldetag: 02.07.2007
(51) Int. Cl.: A61K 36/185, A61P 1/04, A61P 31/04

(54) **Verwendung von Extrakten aus Pelargonium sidoides und/oder Pelargonium reniforme zur Herstellung von Zubereitungen sowie diese Extrakte enthaltende Zubereitungen**

(30) Priorität: 12.07.2006 DE 102006032326
(71) Anmelder: Dr. Willmar Schwabe GmbH & Co. KG, 76227 Karlsruhe (DE)
(72) Erfinder: Beil, Winfried, 30625 Hannover (DE); Hensel, Andreas, 48149 Münster (DE); Koch, Egon, 76229 Karlsruhe (DE); Wittscher, Nicole, 48149 Münster (DE)
(74) Vertreter: Adam, Holger

(57) **Zusammenfassung**

Die Erfindung betrifft die Verwendung von Extrakten aus Pelargonium sidoides und/oder Pelargonium reniforme zur Herstellung von Zubereitungen zur Behandlung oder Prophylaxe von Infektionen mit beta-Lactamase-resistenten Bakterien oder mit Helicobacter pylori sowie deren Folgeerkrankungen, gegebenenfalls auch in Kombination mit beta-Lactamat-Antibiotika, sowie diese Zubereitungen wie Arzneimittel, diätetische Lebensmittel und pharmazeutische Formulierungen, die diese Extrakte enthalten. Des Weiteren betrifft die Erfindung die Verwendung von diätetischen Lebensmitteln zur Behandlung oder Prophylaxe von Infektionen mit beta-Lactamase-resistenten Bakterien oder *Helicobacter pylori* sowie deren Folgeerkrankungen, wobei die diätetischen Lebensmittel Extrakte aus Pelargonium sidoides und/oder Pelargonium reniforme enthalten.

## Beschreibung

Die Erfindung betrifft die Verwendung von Extrakten aus Pelargonium sidoides und/oder Pelargonium reniforme zur Herstellung von Zubereitungen zur Behandlung oder Prophylaxe von Infektionen mit beta-Lactamase-resistenten Bakterien oder mit Helicobacter pylori sowie deren Folgeerkrankungen, gegebenenfalls auch in Kombination mit beta-Lactamat-Antibiotika, sowie diese Zubereitungen wie Arzneimittel, diätetische Lebensmittel und pharmazeutische Formulierungen, die diese Extrakte enthalten. Des Weiteren betrifft die Erfindung die Verwendung von diätetischen Lebensmitteln zur Behandlung oder Prophylaxe von Infektionen mit beta-Lactamase-resistenten Bakterien oder *Helicobacter pylori* sowie deren Folgeerkrankungen, wobei die diätetischen Lebensmittel Extrakte aus Pelargonium sidoides und/oder Pelargonium reniforme enthalten.

Seit der Entdeckung des Bakteriums *Helicobacter pylori* im Jahre 1982 durch Marshall und Warren (Marshall B. J. and Warren J. R., 1984, Lancet, 8390, 1311 - 1315) in Magenschleimhautproben von Patienten haben sich die Ansichten über die Entstehung von Gastritis und Ulcus ventriculi bzw. U. duodeni stark geändert. Lange Zeit ging die Lehrmeinung davon aus, daß diese Magenbeschwerden fast immer eine Folge von übermäßigem Streß und seelischen Belastungen sei. Heute ist klar, dass Magen- und Dünndarmulzera in der Mehrzahl der Fälle durch eine Infektion mit *H*. *pylori* verursacht werden (Stolte M., 1992, Therapiewoche 42, 46, 2726 - 2735).

Die *H. pylori*-Infektion ist weltweit die häufigste Infektion des Menschen, ein Drittel bis die Hälfte der Weltbevölkerung ist mit diesem Keim infiziert. Das wichtigste Erregerreservoir bildet der menschliche Magen, in dem sich auch die Pathologie hauptsächlich abspielt (Rossi R., 1995, Münch. Med. Wschr. 137, 14 - 16). Jede *H*. *pylori*-Infektion löst eine Gastritis (Magenschleimhautentzündung) aus, doch die meisten der chronischen Entzündungen bleiben ohne Symptome. Mehr als 90% aller Zwölffingerdarmgeschwüre (Ulcus duodeni) und 80% aller Magengeschwüre (Ulcus ventriculi) resultieren von einer Infektion mit *H*. *pylori* (Deutsch. Apoth. Ztg. 136, 1996, 4221 - 4223).

Anerkannt ist inzwischen ebenfalls, daß die *H. pylori* bedingte Gastritis eine präkanzeröse Kondition darstellt. Wenn die chronische Magenschleimhautentzündung über längere Zeit fortbesteht, kann es, ähnlich wie bei vielen anderen chronischen Entzündungen, zur Entstehung von Krebs kommen. Gut wissenschaftlich nachgewiesen ist dies insbesondere für eine spezielle Form des Lymphdrüsenkrebses im Magen, dem sogenannten MALT-Lymphom. Als Resultat zahlreicher Studien geht man davon aus, daß *H.pylori*-Infizierte ein drei- bis sechsfach erhöhtes Risiko für die Entstehung eines Magenkarzinoms besitzen. Die WHO hat konsequenterweise *H. pylori* als definitives Kanzerogen eingestuft. Damit allerdings eine Infektion letztlich zu einem bösartigen Tumor führt sind noch weitere Faktoren notwendig, wie z. B. Ernährung und Lebensweise (Hellwig B., 1995, Deutsch. Apoth. Ztg. 135, 38). In etwa 60% der Fälle sind Magenkarzinome mit einer *H. pylori-*Gastritis assoziiert (Stolte M., 1992, Leber Magen Darm, 91-94) und in diesen Fällen kann die Behandlung der chronischen *H. pylori*-Infektion zu einer Heilung des Krebs führen.

Eine Arbeitsgruppe der Deutschen Gesellschaft für Verdauungs- und Stoffwechselkrankheiten hat Leitlinien zur Diagnostik und Therapie der *H*. *pylori*-Infektion erstellt (Caspary W. F. et al., 1996, Leber Magen Darm 26, 301 - 309). Danach werden für einen unkomplizierten *H.pylori* bedingten Ulcus drei verschiedene Therapieschemata empfohlen:
1. Modifizierte ("Italienische") Tripel-Therapie:
   Protonenpumpenhemmer
   Clarithromycin
   Metronidazol
2. Alternative Modifizierte ("Französische") Tripel-Therapie:
   Protonenpumpenhemmer
   Clarithromycin
   Amoxicillin
3. Reserveschema: Quadrupel-Therapie:
   Protonenpumpenhemmer
   Wismutsalz
   Tetrazyklin
   Metronidazol

Die "Italienische" Therapie stellt hinsichtlich Kosten und Effizienz die erste Wahl dar. Durch die Ausbildung von Resistenzen gegenüber Metronidazol kommt es inzwischen aber häufiger zu Therapieversagen (Caspary W. F. et al., 1996, Leber Magen Darm 26, 301 - 309). In diesen Fällen kann die "Französische" Therapie noch erfolgreich sein. Vermehrt wird auch über eine Resistenz von *H. pylori* gegenüber Amoxicillin berichtet. Bei zahlreichen Bakterienarten liegt dieser Resistenz gegenüber Beta-Lactam-Antibiotika, zu denen Amoxicillin gehört, eine Produktion von beta-Lactamase zu Grunde. Häufig wird Amoxicllin deshalb in Kombination mit Clavulinsäure, einem beta-Lactamase-Inhibitor, verabreicht. Auch bei der Behandlung von H. pylori-Infektionen konnte gezeigt werden, dass die Eradikationrate durch diese Kombination verbessert werden kann (Ojetti, V. et al., 2004, J. Intern. Med. 255, 125 - 129). Führt auch die "Französische" Therapie nicht zum Erfolg, so kann noch die Quadrupel-Therapie angewendet werden, die im Prinzip die klassische Tripel-Therapie kombiniert mit einem Wismutsalz darstellt. Allerdings steigt mit jeder der drei Therapieschemata die Nebenwirkungsrate, sie beträgt bei der Quadrupel-Therapie ca. 80%. Insbesondere das Nitroimidazolderivat Metronidazol fällt hier negativ auf, da es als mögliches Karzinogen gilt (The Merck Index, 12th ed., 1996, Nr. 6242).

Es besteht deshalb ein dringendes Bedürfnis nach nebenwirkungsarmen und effektiven Behandlungsmethoden für Infektionen mit beta-Lactamase-resistenten Bakterien und mit Helicobacter pylori. Diese Aufgabe wird gemäß der vorliegenden Erfindung gelöst durch die Verwendung von Extrakten aus Pelargonium sidoides und/oder Pelargonium reniforme, insbesondere aus deren Wurzeln, die entweder alleine, alternativ zu einer in den Therapieschemata genannten Komponenten oder zusätzlich zu diesen eingesetzt werden können.

Extrakte aus den ober- oder unterirdischen Teilen von Pelargonium sidoides und P. reniforme verfügen über eine lange volksmedizinische Anwendung im südlichen Afrika. Die traditionelle Verwendung beinhaltet unter anderem die Behandlung von gastrointestinalen Beschwerden, Dysmenorrhoe und Lebererkrankungen. Die Anwendung bei Magen-Darmerkrankungen beschränkt sich aber vor allem auf die Anwendung bei Durchfällen und wird durch die adstringierende Wirkung auf Grund eines hohen Gerbstoffgehaltes der Extrakte erklärt (Kolodziej, H. und Kayser, O., 1998, Zschr. Phytotherapie 19, 141 - 151). Vorherrschend ist aber die Anwendung von P. sidoides- und P. reniforme-Extrakten bei Atemwegserkrankungen und insbesondere bei Lungentuberkulose.

Seit vielen Jahren haben ethanolisch-wäßrige Extrakte aus Wurzeln von P. sidoides und P. reniforme auch Eingang in den europäischen Arzneischatz gefunden. Ein ethanolischwässiger Extrakt aus den Wurzeln von P. sidoides und P. reniforme zur Behandlung von Infektionen des Respirationstraktes und des Hals-Nasen-Ohren-Bereiches ist seit vielen Jahren unter der Bezeichnung Umckaloabo^{®} auf dem Markt. In pharmakologischen Untersuchungen wurden schwache antimikrobielle Wirkungen sowie immunmodulierende und zytoprotektive Effekte beobachtet. Bei der Testung auf antibakterielle Wirkung wurde deutlich, dass für einen minimalen antimikrobiellen Effekt des Gesamtextraktes grundsätzlich Konzentrationen von 5000 bis 7500 µg/ml erforderlich waren (Kolodziej, H. und Schulz, V., 2003, Dts. Apotheker Ztg. 143, 1303 - 1322). Daneben wird jedoch auch berichtet, dass Extrakte aus Pelargonium sidoides / reniforme abhängig vom gewählten Versuchsmodellen die Adhäsion von A-Streptokokkken an Epithelzellen schon bei Konzentrationen unterhalb von 30 µg/ml sowohl steigern als auch hemmen können (Lallemand, C., et al., 2004, Clin. Microbiol. Infect. 10 (Suppl. 3), 611). Hinweise darauf, dass Extrakte aus P. sidoides oder P. reniforme auch die zelluläre Adhäsion von anderen Bakterien beeinflussen können, gibt es bisher aber ebenso wenig wie Untersuchungen zu möglichen antibakteriellen Wirkungen gegenüber *H. pylori.*

Jetzt wurde überraschend beobachtet, dass Extrakte aus Pelargonium sidoides und Pelargonium reniforme bereits bei Konzentrationen unterhalb von 100 µl/ml nicht nur potent die Adhäsion von H. pylori an Magenepithelzellen hemmen sondern im gleichen Konzentrationsbereich auch schon das Wachstum von H. pylori unterdrücken. Zusätzlich zeigte sich, dass diese Extrakte die Enzymaktivität von beta-Lactamase hemmen und dadurch auch gegen resistente Keime wirksam sind bzw. die Wirkung von beta-Lactamat-Antibiotika verstärken können.

Erfindungsgemäß verwendbare Extrakte aus Pelargonium sidoides und/oder Pelargonium reniforme oder deren Pflanzenteilen können nach bekannten Herstellungsverfahren in variabler Zusammensetzung mit Lösungsmitteln wie z. B. Wasser, Methanol, Ethanol, Aceton, etc., und deren Gemische hergestellt werden. Die Temperaturen können bei Raumtemp. bis 60 °C liegen. Es kann gelinde bis heftig durchmischt werden oder die Extrakte können durch Perkolation innerhalb von 10 Min. bis 24 Std. erhalten werden. Bevorzugte Extraktionslösungsmittel sind dabei Gemische von Ethanol und Wasser, besonders bevorzugt im Verhältnis Ethanol / Wasser = 10/90 bis 12/88 (w/w), insbesondere 11/89 (w/w). Zur Anreicherung wirksamkeitsbestimmender Komponenten (z.B. polymere Polyphenole und Benzopyranone) können weitere Konzentrierungsschritte durchgeführt werden wie z. B. flüssig-flüssig-Verteilung mit z. B. 1-Butanol/Wasser oder Ethylacetat/Wasser, Adsorption-Desorption an lonenaustauscher, LH20, HP20 und andere Harze oder chromatographische Abtrennungen über RP18, Kieselgel, etc.. Falls die Weiterverarbeitung zu Trockenextrakten erwünscht ist, erfolgt diese nach an sich bekannten Verfahren durch Abziehen des Lösungsmittels bei erhöhter Temperatur und / oder reduziertem Druck oder durch Gefriertrocknung.

Bei den erfindungsgemäß verwendeten Extrakten aus Pelargonium sidoides und Pelargonium reniforme handelt es sich entsprechend Ph. Eur. um flüssige, halbfeste oder Trockenextrakte. Diese können in Form von flüssigen oder festen pharmazeutischen Darreichungsformen angewendet werden. Vorzugsweise werden Darreichungsformen angewendet, bei denen der Extrakt entweder bereits in gelöster Form vorliegt (z.B. Tropflösungen, Sirupe, aufgelöste Brausetabletten) oder bei denen der Extrakt nach der peroralen Applikation im Magen freigesetzt wird (z.B. Tabletten, überzogene Tabletten oder Kapseln). Eine weitere bevorzugte Darreichungsform für die Extrakte aus P. sidoides und P. reniforme sind Tabletten zur Anwendung in der Mundhöhle, bei denen der Wirkstoff im Mund über eine gewisse Zeitspanne freigesetzt und kontinuierlich geschluckt wird (z.B. Lutschtabletten, Kautabletten).

Die erhaltenen Extrakte können zusammen mit üblichen pharmazeutischen Hilfsstoffen zu den genannten pharmazeutischen Zubereitungen wie Kapseln, Tabletten, überzogenen Tabletten verarbeitet werden. Als pharmazeutische Hilfsstoffe werden Füll-, Binde-, Spreng-, Schmier- und Überzugsmittel für Tabletten und überzogene Tabletten verwendet. Alternativ können die Extrakte in pharmazeutisch verwendeten Lösungsmitteln wie Wasser, Ethanol und Glycerol aufgelöst werden.

Zur Herstellung von Tabletten wird der Extrakt mit geeigneten pharmazeutisch verträglichen Hilfstoffen wie z. B. Laktose, Cellulose, Siliciumdioxid, Croscarmellose und Magnesiumstearat gemischt und zu Tabletten gepresst, die gegebenenfalls mit einem geeigneten Überzug z. B. aus Hydroxymethylpropylcellulose, Polyethylenglykol, Farbstoffen (z. B. Titandioxid, Eisenoxid) und Talkum versehen werden.

Die erfindungsgemäßen Extrakte können auch, ggf. unter Zusatz von Hilfstoffen wie z. B. Stabilisatoren, Füllmittel etc., in Kapseln abgefüllt werden.

Die Dosierung erfolgt dabei so, dass pro Tag 2 bis 1000 mg, bevorzugt 10 bis 200 mg Trockenextrakt zugeführt werden.

Die Wirksamkeit von Extrakten aus Pelargonium sidoides und/oder Pelargonium reniforme bei Infektionen mit beta-Lactamase-resistenten Bakterien und *H. pylori* wird durch die nachstehenden Versuche belegt.

Figur 1 zeigt die Wirkung einer Lösung eines Trockenextrakts aus Wurzeln von Pelargonium sidoides und Pelargonium reniforme auf das Wachstum von *H. pylori.*

Figur 2 zeigt die Wirkung des Trockenextrakts aus Wurzeln von Pelargonium sidoides auf das Wachstum von *H. pylori* in Kokultur mit Magen-AGS-Zellen.

Figur 3 zeigt die Hemmung der Adhärenz von *H. pylori* an AGS Zellen durch eine Lösung eines Trockenextrakts aus Wurzeln von Pelargonium sidoides / reniforme.

Figur 4 zeigt die Hemmung der beta-Lactamase durch einen Trockenextrakt aus Wurzeln von Pelargonium sidoides (Extraktionsmittel Wasser / Ethanol 89/11 w/w).

Zur Untersuchung der Wirkung von Extrakten aus Pelargonium sidoides und Pelargonium reniforme auf das Wachstum von *H. pylori* wurden zwei klinische Isolate verwendet (H.p. 39 und H.p. 4). Bei H.p. 4 und 39 handelt es sich um CagA-negative und nichtzytotoxinproduzierende Stämme. Die Keime wurden auf Columbia Agarplatten mit 5 % Schafblut unter mikroaerophilen Bedingungen (5 % O₂, 10 % CO₂, 85 % N₂) bei 37 °C 48 Std. angezüchtet. Die Keime wurden in BHI-Medium geerntet und die optische Dichte auf 1,0 (ca. 1,5 x 10⁸ CFU) eingestellt.

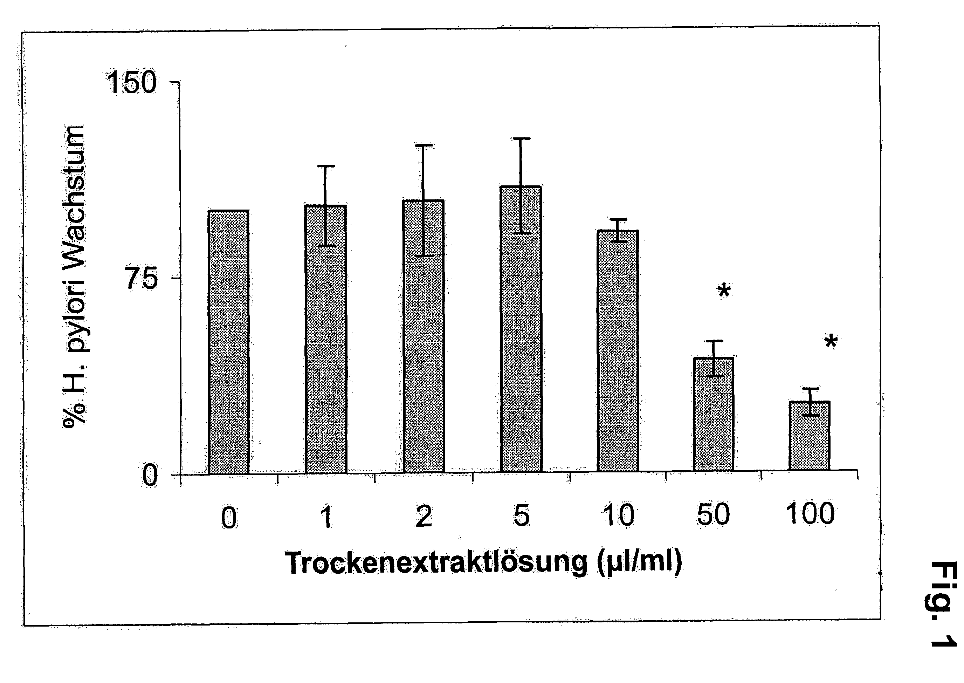

Zur Prüfung der antibakteriellen Wirkung von Extrakten aus Pelargonium sidoides und Pelargonium reniforme auf *H*. *pylori* wurden 1,5 x 10⁷ *H*. *pylori* (*OD:* 0,1) in 1 ml BHI-Medium plus 10% FCS in 24-well Platten ausgesät und 48 Std. unter mikroaerophilen Bedingungen mit ansteigenden Konzentrationen (1- 100 µl/ml entsprechend ca. 8 - 800 µg/ml Trockenextrakt) Lösung eines Trockenextrakts aus Wurzeln von Pelargonium sidoides / reniforme (Extraktionsmittel Wasser / Ethanol 89/11 w/w) inkubiert. Die OD von *H. pylori* wurde nach 48 Std.-Inkubation bestimmt. Wie aus der Figur 1 ersichtlich ist hemmte die Extraktlösung konzentrationsabhängig das Wachstum von *Helicobacter pylori.* Angegeben sind die Mittelwerte ± SE von drei unabhängigen Versuchen mit den Stämmen H.p. 39 und H.p. 4.

In einem zweiten Versuchsansatz wurde die Wirkung eines Trockenextrakts aus Wurzeln von Pelargonium sidoides (Extraktionsmittel Wasser / Ethanol 89/11 w/w) auf das Wachstum von adhärenten *H. pylori* in Kokultur mit Magen-AGS-Zellen untersucht. 10.000 AGS-Zellen wurden in 96-well Platten in 0,2 ml RPMI Medium mit 10 % FCS 24 Std. bei 37 0C (5 % CO₂, Luft, Luftfeuchtigkeit: 98 %) inkubiert. Der Zellmonolayer wurde mit 10⁵ *H. pylori* infiziert.

Nach 3 Std. erfolgte ein Mediumwechsel und die Zugabe des Extrakts. Nach 24 Std. Inkubation wurde das Medium abgesaugt und die *H. pylori* Dichte mittels Urease-Test (Medium: 0,2 ml 0,9 % NaCl, 1 mmol/L Citrat pH 7,4, 20 mmol/L Harnstoff, 14 µg/ml Phenolrot) bestimmt.

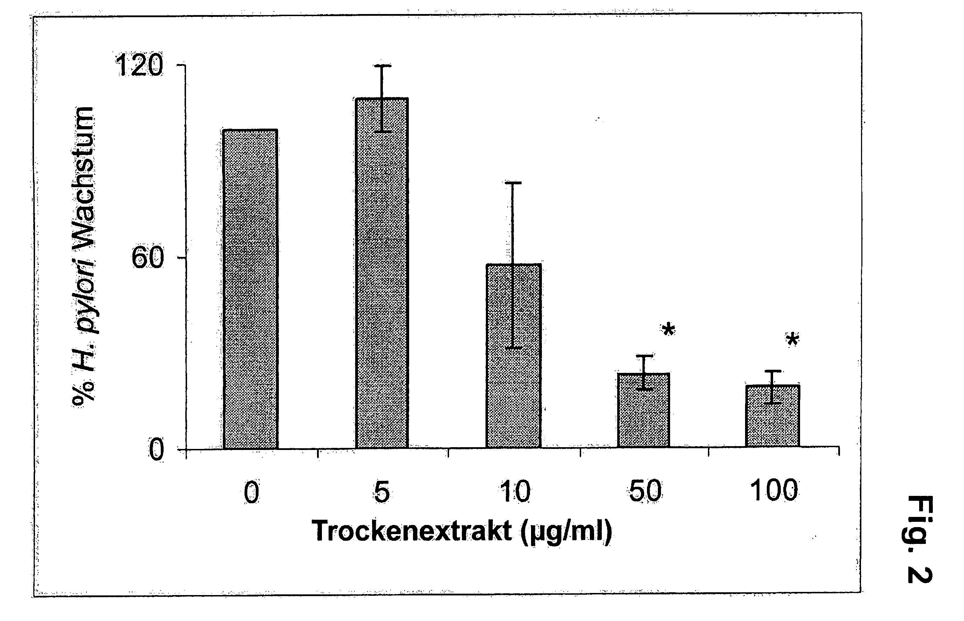

In den Kokulturexperimenten waren 24 Std. nach initialer Infektion mit 10⁵ *H. pylori* 6,6 x 10⁵ Keime nachweisbar. Der Trockenextrakt aus Wurzeln von Pelargonium sidoides hemmte das Wachstum von *H. pylori* dosisabhängig. Das Wachstum der *H. pylori-Stämme* H.p. 39 und H.p. 4 wurde durch 100 pg/ml um ca. 90 % reduziert. (Figur 2). Angegeben ist der Mittelwert ± SE von drei unabhängigen Versuchen mit dem Stamm H.p. 4. p <0,05 vs. Kontrolle.

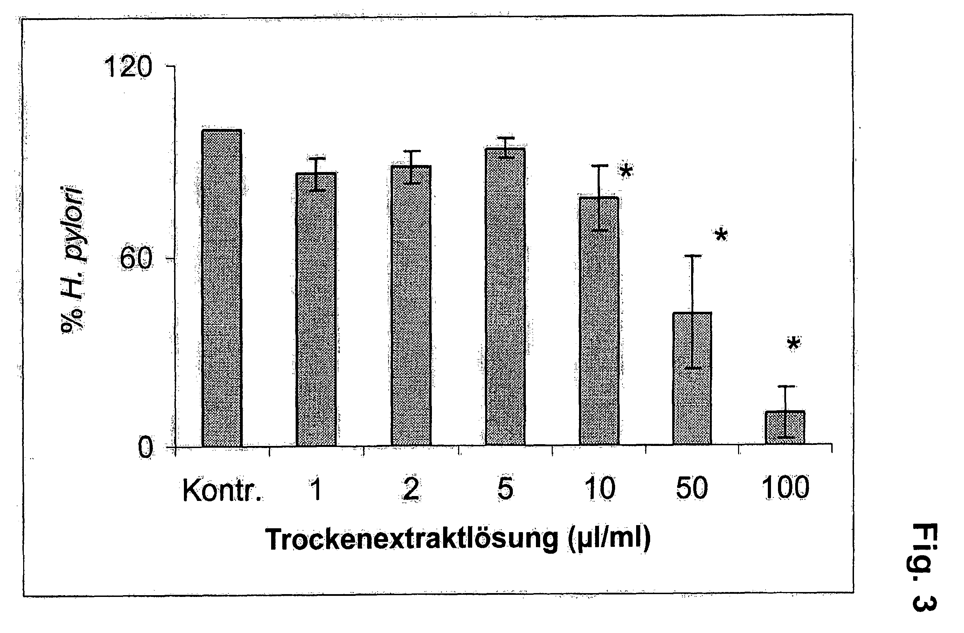

Zur Prüfung der Frage, ob eine Lösung eines Trockenextrakts aus Wurzeln von Pelargonium sidoides / reniforme neben seiner bakterioziden/bakteriostatischen Wirkung auch die Anlagerung von *H. pylori* an Magenepithelzellen hemmt, wurden Magenepithelzellmonolayer (AGS-Zellen) über drei Stunden mit dem Keim (10⁵ *H. pylori*) in Anwesenheit einer Lösung eines Trockenextrakts aus Wurzeln von Pelargonium sidoides / reniforme (Extraktionsmittel Wasser / Ethanol 89/11 w/w) koinkubiert. Danach wurden die nicht-adhärenten Keime und der Extrakt ausgewaschen und die Keimdichte nach einer Inkubation von 21 Std. mittels Urease-Aktivität bestimmt. Wie aus der Figur 3 hervorgeht, hemmt der Extrakt konzentrationsabhängig (1 - 100 µl/ml entsprechend ca. 8 - 800 µg/ml Trockenextrakt) die Adhärenz von *H. pylori-Stämmen* an AGS-Zellen, wobei für beide Isolate weitgehend gleiche Effekte beobachtet wurden. Angegeben sind Mittelwerte ± SE mit dem *H. pylori* Stamm 39. p < 0,05 vs. Kontrolle.

Zur Prüfung auf Hemmung der beta-Lactamase (Penicillinase) wurde gereinigtes Enzym (Sigma, Taufkirchen , Nr. P-0389) verwendet. Beta-Lactamase (0,5 U/ml) wurde in Müller-Hinton-Puffer (Sigma) mit 25 mg/l Ca⁺⁺ und 12,5 mg/l Mg⁺⁺ gelöst und in einem Volumen von 100 µl zusammen mit unterschiedlichen Konzentrationen eines Trockenextrakts aus Wurzeln von Pelargonium sidoides (Extraktionsmittel Wasser / Ethanol 89/11 w/w) für 18 h bei 37 °C in einer 96-Well F-Form Mikrotiterplatte inkubiert. Als Substrat wurde Nitrocefin (20 µg/ml) in 0,1 M Phosphatpuffer (pH 7,0) mit Zusatz von 5 % DMSO verwendet. Nitrocefin (Oxoid, Wesel) ist ein chromogenes Cephalosporin-Derivat. Nach enzymatischer Spaltung der Amidbindung im beta-Lactamring durch beta-Lactamase kommt es zu einer charakteristischen Rotfärbung, die photometrisch bei einer Wellenlänge von 490 nm gemessen werden kann. Die Messung der Enzymreaktion erfolgte kinetisch über einen Zeitraum von 10 min. Die Hemmung der Enzymaktivität wurde im Vergleich zu einer paralell untersuchten Lösungsmittelkontrolle bestimmt. Als Referenzsubstanz wurde Clavulansäure (Wako Chemicals GmbH, Neuss) verwendet.

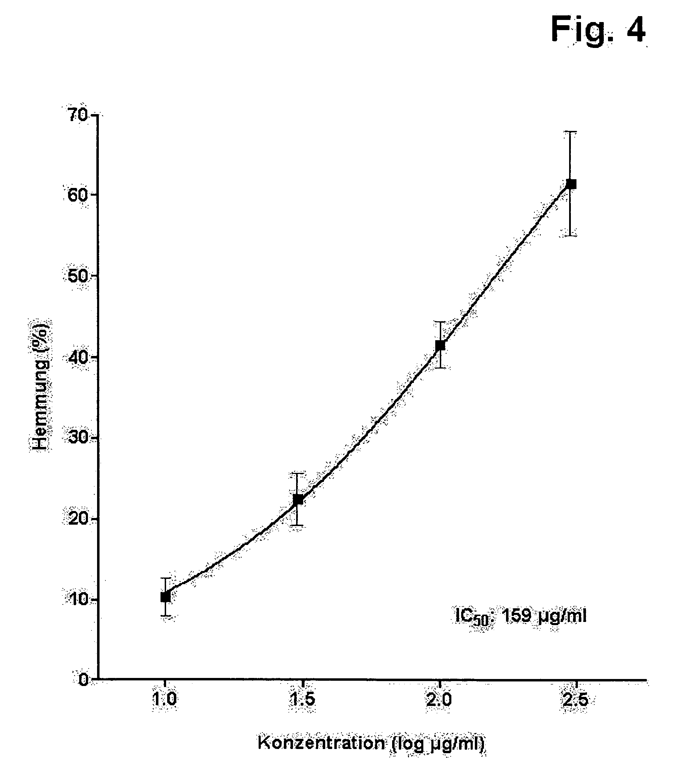

Der Extrakt bewirkte konzentrationsabhängig eine Hemmung der beta-Lactamase (Figur 4) mit einer halbmaximalen Hemmkonzentration von 159 µg/ml. Im Vergleich dazu hemmte Clavulansäure die Enzymaktivität bei einer Konzentration von 30 µg/ml um ca. 70 %.

## Patentansprüche

1. Verwendung von Extrakten aus Pelargonium sidoides und/oder Pelargonium reniforme zur Herstellung einer Zubereitung zur Behandlung oder Prophylaxe von Infektionen mit beta-Lactamase-resistenten Bakterien oder mit Helicobacter pylori sowie deren Folgeerkrankungen.

2. Verwendung nach Anspruch 1, wobei die Extrakte aus Wurzeln von Pelargonium sidoides und/oder Pelargonium reniforme hergestellt werden.

3. Verwendung nach einem der Ansprüche 1 oder 2, wobei der Extrakt ein Trockenextrakt ist.

4. Verwendung nach einem der Ansprüche 1 oder 2, wobei der Extrakt ein Flüssigextrakt ist.

5. Verwendung nach einem der Ansprüche 1 bis 4, wobei der Extrakt einen oder mehrere angereicherte Inhaltsstoffe aus Pelargonium sidoides und/oder Pelargonium reniforme enthält.

6. Verwendung nach Anspruch 5, wobei die angereicherten Inhaltsstoffe ausgewählt sind aus polymeren Polyphenolen und Benzopyranonen.

7. Verwendung nach einem der Ansprüche 1 bis 6, wobei der Extrakt aus Pelargonium sidoides und/oder Pelargonium reniforme in Kombination mit beta-Lactamat-Antibiotika eingesetzt wird.

8. Verwendung nach einem der Ansprüche 1 bis 7, wobei die Folgeerkrankungen einer Helicobacter pylori-Infektion ausgewählt sind aus Gastritis, Zwölffingerdarmgeschwür, Magengeschwür, MALT-Lymphom und Magenkarzinom.

9. Verwendung nach einem der Ansprüche 1 bis 8, wobei der Extrakt oral verabreicht wird.

10. Verwendung nach Anspruch 9, wobei der Extrakt in Form einer Lösung oder eines Sirups verabreicht wird.

11. Verwendung nach Anspruch 9, wobei der Extrakt in Form einer Tablette, überzogenen Tablette oder Kapsel verabreicht wird.

12. Verwendung nach Anspruch 9, wobei der Extrakt in Form einer Lutschtablette oder Kautablette verabreicht wird.

13. Verwendung nach einem der Ansprüche 1 bis 12, wobei die Zubereitung ein Arzneimittel ist.

14. Verwendung nach einem der Ansprüche 1 bis 12, wobei die Zubereitung ein diätetisches Lebensmittel ist.

15. Verwendung eines diätetischen Lebensmittels zur Behandlung oder Prophylaxe von Infektionen mit beta-Lactamase-resistenten Bakterien oder mit Helicobacter pylori sowie deren Folgeerkrankungen, wobei das diätetische Lebensmittel einen oder mehrere Extrakte aus Pelargonium sidoides und/oder Pelargonium reniforme enthält.

16. Verwendung nach Anspruch 15, wobei die Extrakte aus Wurzeln von Pelargonium sidoides und/oder Pelargonium reniforme hergestellt werden.

17. Verwendung nach einem der Ansprüche 15 oder 16, wobei der Extrakt ein Trockenextrakt ist.

18. Verwendung nach einem der Ansprüche 15 oder 16, wobei der Extrakt ein Flüssigextrakt ist.

19. Verwendung nach einem der Ansprüche 15 bis 18, wobei der Extrakt einen oder mehrere angereicherte Inhaltsstoffe aus Pelargonium sidoides und/oder Pelargonium reniforme enthält.

20. Verwendung nach Anspruch 19, wobei die angereicherten Inhaltsstoffe ausgewählt sind aus polymeren Polyphenolen und Benzopyranonen.

21. Verwendung nach einem der Ansprüche 15 bis 20, wobei der Extrakt aus Pelargonium sidoides und/oder Pelargonium reniforme in Kombination mit beta-Lactamat-Antibiotika eingesetzt wird.

22. Verwendung nach einem der Ansprüche 15 bis 21, wobei die Folgeerkrankungen einer Helicobacter pylori-Infektion ausgewählt sind aus Gastritis, Zwölffingerdarmgeschwür, Magengeschwür, MALT-Lymphom und Magenkarzinom.

23. Verwendung nach einem der Ansprüche 15 bis 22, wobei der Extrakt oral verabreicht wird.

24. Verwendung nach Anspruch 23, wobei der Extrakt in Form einer Lösung oder eines Sirups verabreicht wird.

25. Verwendung nach Anspruch 23, wobei der Extrakt in Form einer Tablette, überzogenen Tablette oder Kapsel verabreicht wird.

26. Verwendung nach Anspruch 23, wobei der Extrakt in Form einer Lutschtablette oder Kautablette verabreicht wird.

27. Zubereitung zur Behandlung oder Prophylaxe von Infektionen mit beta-Lactamase-resistenten Bakterien oder mit Helicobacter pylori sowie deren Folgeerkrankungen, **gekennzeichnet durch** einen Gehalt an einem Extrakt aus Pelargonium sidoides und/oder Pelargonium reniforme.

28. Zubereitung nach Anspruch 27, in dem zusätzlich ein beta-Lactamat-Antibiotikum enthalten ist.

29. Zubereitung nach einem der Ansprüche 27 oder 28 zur oralen Verabreichung.

30. Zubereitung in Form einer Lösung oder eines Sirups, enthaltend einen oder mehrere Extrakte, wie in Anspruch 1 bis 7 definiert.

31. Zubereitung in Form einer Tablette, überzogenen Tablette oder Kapsel, enthaltend einen oder mehrere Extrakte, wie in Anspruch 1 bis 7 definiert.

32. Zubereitung in Form einer Lutschtablette oder Kautablette enthaltend einen oder mehrere Extrakte, wie in Anspruch 1 bis 7 definiert.

33. Zubereitung nach einem der Ansprüche 27 bis 32, wobei die Zubereitung ein Arzneimittel ist.

34. Zubereitung nach einem der Ansprüche 27 bis 32, wobei die Zubereitung ein diätetisches Lebensmittel ist.

35. Zubereitung nach einem der Ansprüche 27 bis 32, wobei die Zubereitung eine pharmazeutische Formulierung ist.
